# FASCICULE DE BREVET EUROPEEN

(11) **EP 2 029 133 B1**
(45) Date de publication et mention de la délivrance du brevet: **05.11.2014**
(21) Numéro de dépôt: 07766126.2
(22) Date de dépôt: 31.05.2007
(51) Int. Cl.: A61K 31/192, A61K 47/30, A61K 47/36, A61K 47/38, A61K 9/10, A61P 17/00, A61P 17/10, A61K 8/04, A61K 8/368, A61K 8/72, A61K 8/73, A61Q 5/00, A61Q 19/00, A61Q 19/08, A61Q 17/04

(54) **COMPOSITIONS COMPRENANT AU MOINS UN DÉRIVÉ DE L'ACIDE NAPHTOÏQUE ET AU MOINS UN AGENT FILMOGÈNE, LEURS PROCÉDÉS DE PRÉPARATION, ET LEURS UTILISATIONS**
ZUSAMMENSETZUNGEN MIT MINDESTENS EINEM NAPHTHOESÄUREDERIVAT UND MINDESTENS EINEM FILMBILDENDEN MITTEL, HERSTELLUNGSVERFAHREN UND VERWENDUNG
COMPOSITIONS COMPRISING AT LEAST ONE NAPHTHOIC ACID DERIVATIVE AND AT LEAST ONE FILM-FORMING AGENT, METHODS FOR PREPARING SAME, AND USES THEREOF

(30) Priorité: 31.05.2006 FR 0651982
(43) Date de publication de la demande: 04.03.2009
(73) Titulaire: Galderma Research & Development, 06410 Biot (FR)
(72) Inventeur: MALLARD, Claire, F-06250 Mougins (FR); FERRARA, Eve, F-06560 VALBONNE (FR)
(74) Mandataire: Starck-Loudes, Anne-Caroline
(86) Numéro de dépôt international: PCT/FR2007/051359
(87) Numéro de publication internationale: WO 2007/138231

(56) Documents cités:
- EP-A1- 0 199 636
- WO-A-95/04517
- WO-A2-2004/052353
- FR-A- 2 871 377
- FR-A1- 2 837 101
- "Hyaluronic Acid (HA) for Skin and Joints" DISCOUNT VITAMINS & HERBS, [Online] 30 mai 2006 (2006-05-30), pages 1-5, XP002415428 Extrait de l'Internet: URL:http://web.archive.org/web/20060530003 853/http://www.discount-vitamins-herbs.net /Hyaluronic-acid.htm> [extrait le 2007-01-17]

## Description

La présente invention se rapporte à des compositions pour application topique, des procédés de préparation de telles compositions, et leurs utilisations en tant que produits cosmétiques ou pharmaceutiques, lesdites compositions étant destinées, en particulier, au traitement de l'acné.

L'acné est une pathologie multifactorielle fréquente qui atteint la peau riche en glandes sébacées (visage, région scapulaire, bras et régions intertrigineuses). Elle est la plus fréquente des dermatoses. Les cinq facteurs pathogéniques suivants jouent un rôle déterminant dans la constitution de l'acné :
1. la prédisposition génétique;
2. la surproduction de sébum (séborrhée);
3. les androgènes;
4. les troubles de la kératinisation folliculaire (comédogenèse); et
5. la colonisation bactérienne et les facteurs inflammatoires.

Il existe plusieurs formes d'acnés, ayant toutes en commun l'atteinte des follicules pilosébacés. On peut citer notamment, l'acné conglobata, l'acné chéloïde de la nuque, l'acné médicamenteuse, l'acné miliaire récidivante, l'acné nécrotique, l'acné neonatorum, l'acné prémenstruelle, l'acné professionnelle, l'acné rosacée, l'acné sénile, l'acné solaire, et l'acné vulgaire.

L'acné vulgaire, appelée également acné juvénile polymorphe, est la plus courante. Elle comprend quatre stades, mais le passage par tous les stades n'est pas obligatoire :
- Le stade 1 correspond à l'acné comédonienne caractérisée par un grand nombre de comédons ouverts et/ou fermés, et de microkystes.
- Le stade 2, ou acné papulopustuleuse, est de gravité légère à modérée. Elle est caractérisée par la présence de comédons ouverts et/ou fermés, de microkystes, mais également de papules rouges et de pustules. Elle touche principalement le visage et laisse peu de cicatrices.
- Le stade 3, ou acné papulocomédonienne, est plus grave et s'étend au dos, au thorax et aux épaules. Elle est accompagnée d'un plus grand nombre de cicatrices.
- Le stade 4, ou acné nodulokystique, s'accompagne de nombreuses cicatrices. Elle présente des nodules ainsi que des pustules volumineuses violacées et douloureuses.

Les différentes formes d'acné décrites précédemment peuvent être traitées par des actifs tels que les anti-séborrhéiques et les anti-infectieux, par exemple le peroxyde de benzoyle (notamment le produit Eclaran® commercialisé par la société Pierre Fabre), par des rétinoïdes tels que la trétinoïne (notamment le produit Retacnyl® commercialisé par la société Galderma) ou l'isotrétinoïne (produit Roaccutane® commercialisé par les Laboratoires Roche), ou par des dérivés d'acide naphtoïque. Les dérivés d'acide naphtoïque tels que notamment l'acide 6-[3-(1-adamantyl)-4-méthoxyphényl]-2-naphtoïque), communément appelé adapalène (produit Differine® commercialisé par la société Galderma), sont largement décrits et reconnus comme des principes actifs aussi efficaces que la trétinoïne pour le traitement de l'acné. L'adapalène présente en outre l'avantage de provoquer moins d'effets secondaires, tels des phénomènes d'irritation, d'assèchement de la peau ou d'intolérance, que les autres actifs décrits précédemment, ce qui en fait un produit de choix. L'adapalène a ainsi été utilisée dans des compositions dépigmentantes se présentant sous la forme de gels hydroalcooliques comprenant un filtre solaire et un dérivé phénolique (WO 2004/052353) ou du méquinol (FR 2 871377).

Cependant, l'homme de l'art cherche en permanence à améliorer l'efficacité et la tolérance des compositions contenant ce type d'actif. Une des solutions pour améliorer l'efficacité est d'augmenter les quantités d'actifs présents dans la composition ou d'augmenter les durées de traitement. De telles modifications entraînent généralement une augmentation de l'irritation induite. C'est pourquoi il est nécessaire de réaliser des compositions pouvant encore améliorer la tolérance des principes actifs.

Un problème que se propose de résoudre l'invention est de réaliser des compositions stables et moins irritantes que celles de l'art antérieur. De telles compositions doivent de plus favoriser la pénétration topique du principe actif sous forme dispersée.

Il se trouve que la demanderesse a mis en évidence de manière surprenante que des ingrédients connus pour conférer à une composition un effet filmogène peuvent également améliorer la tolérance de principes actifs irritants, tels que les actifs anti-acnéiques et notamment les dérivés de l'acide naphtoïque, tel l'adapalène.

Ainsi, un des buts de la présente invention est de proposer une composition pour application topique particulièrement efficace, comprenant un dérivé d'acide naphtoïque, sans présenter d'effet manifestement irritant empêchant son utilisation à plus ou moins long terme par le sujet.

L'invention a pour premier objet une composition pour application topique comprenant, dans un milieu physiologiquement acceptable, au moins un dérivé de l'acide naphtoïque et au moins un agent filmogène, ledit dérivé d'acide naphtoïque étant sous une forme dispersée dans ladite composition et ayant la formule (I) telle que définie dans la revendication 1.

Par actif sous forme dispersée selon l'invention, on entend un principe actif sous forme de particules solides, mises en suspension dans un véhicule donné. De telles particules ont notamment une taille supérieure à 10 µm.

Elle a pour deuxième objet un procédé de préparation d'une composition pour application topique, caractérisé en ce qu'il comprend l'étape de mélange d'un véhicule physiologiquement acceptable comprenant au moins un dérivé de l'acide naphtoïque avec au moins un agent filmogènes, ledit dérivé d'acide naphtoïque étant sous une forme dispersée dans ladite composition et ayant la formule (I) telle que définie dans la revendication 1. Par véhicule physiologiquement acceptable, on entend un véhicule compatible avec la peau, les muqueuses et/ou les phanères.

Enfin, elle a pour troisième objet l'utilisation d'une composition telle que décrite ci-dessus pour la préparation d'un médicament destiné au traitement et/ou à la prévention des affections dermatologiques liées à un désordre de la kératinisation portant sur la différenciation et sur la prolifération cellulaire, et notamment pour prévenir et/ou traiter les acnés comédoniennes, vulgaires, papulocomédoniennes, nodulokystiques, les acnés polymorphes, les acnés rosacées, les acnés conglobata, les acnés séniles, ou encore les acnés secondaires telles que l'acné solaire, médicamenteuse ou professionnelle.

Lorsqu'une composition comprend, dans un milieu physiologiquement acceptable, au moins un dérivé de l'acide naphtoïque et au moins un agent filmogène, ledit dérivé d'acide naphtoïque étant sous une forme dispersée dans ladite composition, elle présente une très bonne tolérance sans modifier la quantité d'actif ayant pénétré dans peau.

La composition selon l'invention comprend au moins un dérivé de l'acide naphtoïque et au moins un agent filmogène selon la revendication 1.

L'acide naphtoïque est un composé de formule :

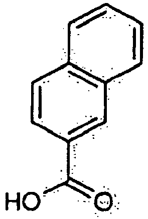

Par dérivé de l'acide naphtoïque, on entend les composés de formule (I): 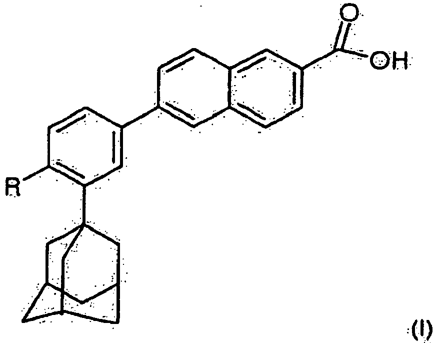 où R représente un atome d'hydrogène, un radical hydroxyle, un radical alkyle, ramifié ou non, ayant de 1 à 4 atomes de carbone, un radical alkoxy ayant de 1 à 10 atomes de carbone ou un radical cycloaliphatique substitué ou non.

Par radical alkyle linéaire ou ramifié ayant de 1 à 4 atomes de carbone, on entend de préférence les radicaux méthyle, éthyle, propyle et butyle.

Par radical alkoxy ayant de 1 à 10 atomes de carbone, on entend de préférence les radicaux méthoxy, éthoxy, propoxy, butoxy, hexyloxy et décyloxy.

Par radical cycloaliphatique, on entend de préférence les radicaux mono ou polycyclique tel que le radical methyl-1 cyclohexyle ou le radical 1-adamantyle.

Parmi les dérivés de l'acide naphtoïque susceptibles d'entrer dans les compositions selon l'invention, on choisira avantageusement l'acide 6-[3-(1-adamantyl)-4-methoxyphenyl]-2-naphtoïque (adapalène), l'acide 6-[3-(1-adamantyl)-4-hydroxyphenyl]-2-naphtoïque, l'acide 6-[3-(1-adamantyl)-4-decyloxyphenyl]-2-naphtoïque et l'acide 6-[3-(1-adamantyl)-4-hexyloxyphenyl]-2-naphtoïque.

Les dérivés de l'acide naphtoïque précités se présentent généralement sous une forme dispersée dans la composition selon l'invention. Les dérivés de l'acide naphtoïque insolubles sont ainsi répartis de façon homogène dans la composition selon l'invention.

Dans les compositions selon l'invention, les dérivés de l'acide naphtoïque sont utilisés à des concentrations inférieures ou égales à 10 % en poids par rapport au poids total de la composition, et de préférence sont comprises entre 0,001% et 10% en poids par rapport au poids total de la composition et, préférentiellement entre 0,01 % et 5%, plus préférentiellement, entre 0,05% et 2%, et tout préférentiellement de 0,1% à 0,3% en poids par rapport au poids total de la composition.

Dans l'ensemble du présent texte, à moins qu'il ne soit spécifié autrement, il est entendu que lorsque des intervalles de concentrations sont donnés, ils incluent les bornes supérieures et inférieures dudit intervalle.

De façon avantageuse, le dérivé de l'acide naphtoïque utilisé dans les compositions selon l'invention est l'adapalène. La concentration de l'adapalène utilisée dans la composition selon l'invention est alors comprise entre 0,01% et 0,5%, plus préférentiellement entre 0,1% et 0,3%, en particulier à une concentration d'environ 0,1% ou à une concentration d'environ 0,3%.

La composition selon l'invention comprend en outre au moins un agent filmogène tel que défini dans la revendication 1.

Par agent filmogène, on entend un polymère hydrophile de masse moléculaire au moins supérieure à 10000, qui, lors de l'application sur la peau, forme un film. La demanderesse a mis en évidence que ces agents filmogènes, outre des propriétés gélifiantes, confèrent une meilleure tolérance à la composition les contenant.

A titre d'exemple non limitatif d'agents filmogènes, on peut citer les polyvinylpyrrolidones, de préférence hydrosolubles, soit la povidone comme les Kollidon® et les dérivés acétates de vinyle comme la copovidone, les polysaccharides, les alcools polyvinyliques, les celluloses et dérivés, les polymères cyanoacryliques ou encore les polyacrylamides, les copolymères acryliques, acryliques/méthacryliques et polyméthacrylate/butylacrylate, acrylique/acrylate. L'agent filmogène hydrosoluble utilisé selon l'invention peut être d'origine naturelle, comme le hyaluronate de sodium.

Parmi les polyvinylpyrrolidones et dérivés, on peut citer le poly-1-vinyl-2-pyrrolidone, encore appelé povidone, ou le copolymère polyvinylpyrrolidone/vinyl acétate, encore appelé copovidone, comme le Kollidon® VA64.

Comme exemple de polysaccharides, on peut citer les celluloses et dérivés comme la carboxyméthylcellulose ou encore l'hydroxypropylcellulose, l'hydroxyéthylcellulose. Parmi les autres polysaccharides, on peut également citer les gommes comme la gomme xanthane, la gomme guar, la gomme karaya, les gommes carrhagénanes, les pectines, le hyaluronate de sodium.

De façon préférentielle, l'agent filmogène hydrosoluble, selon l'invention est choisi parmi les polyvinylpyrrolidones et leurs copolymères solubles, comme par exemple la copovidone et parmi les polysaccharides comme le hyaluronate de sodium.

Dans les compositions selon l'invention, les agents filmogènes sont utilisés à des concentrations inférieures ou égales à 20%, de préférence comprises entre 0,5% et 20% en poids par rapport au poids total de la composition et, plus préférentiellement, comprises entre 1% et 10% et en particulier 2%, 4%, ou 6%.

La présence d'au moins un agent filmogène permet d'améliorer la tolérance et présente donc un effet particulièrement intéressant dans le cas des formulations comprenant de l'adapalène. En effet, les dérivés d'acide naphtoïque peuvent être irritants et présenter une action desséchante sur la peau. Il est donc intéressant de diminuer l'irritation induite afin de pouvoir augmenter les doses.

Les compositions de la présente invention peuvent se présenter sous toutes les formes galéniques normalement utilisées pour une application topique, notamment sous forme de dispersions aqueuses, hydroalcooliques ou huileuses, de dispersions du type lotion, de gels aqueux, anhydres ou lipophiles, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-liquide ou solide du type crème, gel-crème ou pommade ou encore de micro émulsions, de micro capsules, de micro particules ou de dispersions vésiculaires de type ionique et/ou non ionique.

De préférence, les compositions selon l'invention se présentent sous forme de lotions, de gels-crèmes, de gels ou de crèmes, et plus préférentiellement sous la forme de gels.

L'homme du métier veillera à choisir les excipients constituant les compositions selon l'invention en fonction de la forme galénique souhaitée et de manière à ce que les propriétés avantageuses de la composition selon l'invention soient respectées.

La composition selon l'invention peut en outre notamment comprendre un ou plusieurs des ingrédients suivants :
a) un ou plusieurs agents gélifiants ou agents de suspension,
b) un ou plusieurs agents chélatants,
c) un ou plusieurs agents émollients ;
d) un ou plusieurs agents mouillants,
e) un ou plusieurs agents conservateurs.

A titre d'exemple non limitatif de gélifiants ou agents de suspension pouvant entrer dans les compositions selon l'invention, on peut citer les carbomers vendus sous le nom générique de Carbopol®, les carbomers dits non sensibles aux électrolytes, vendus sous le nom d'Ultrez 10® ou de Carbopol ETD® par la société BF Goodrich, les polysaccharides avec à titre d'exemples non limitatifs la gomme de xanthane telle que le Keltrol T® vendu par la société Kelco, la gomme guar, les chitosans, la cellulose et ses dérivés tel que l'hydroxyéthylcellulose, en particulier, le produit vendu sous le nom de Natrosol HHX 250® par la société Aqualon, et le copolymère d'acrylamide de sodium et d'acrylamino-2-méthylpropane sulphonate en dispersion à 40% dans l'isohéxadécane et le polysorbate 80 vendu sous le nom de Simulgel 600® par la société Seppic.

A titre de gélifiant préféré, on peut citer les carbomers vendus notamment sous les noms Carbopol 974P NF et Carbopol 980 NF.

Parmi les agents chélatants, on peut citer à titre d'exemples non limitatifs l'acide éthylène diamine tetraacétique (EDTA), l'acide diéthylène triamine pentaacétique (DTPA), l'acide éthylène diamine-di (O-hydroxyphényl acétique) (EDDHA), l'acide hydroxy-2-éthylène diamine triacétique (HEDTA), l'acide éthyldiamine-di (O-hydroxy-p-méthyl phényl) acétique (EDDHMA) et l'acide éthylène diamine-di (5-carboxy-2-hydroxyphényl) acétique (EDDCHA).

A titre d'agent chélatant préféré, on peut citer l'acide éthylène diamine tetraacétique (EDTA) vendu notamment sous le nom Titriplex III®.

Parmi les agents émollients qui ont pour rôle d'hydrater la peau et de faciliter l'application de la formulation, on utilise préférentiellement, sans que cette liste soit limitative, des composés tels que la glycérine, le propylène glycol, le dipropylène glycol, le propylène glycol dipélargonate, le lauroglycol, seuls ou en mélange.

A titre d'agent émollient préféré, on peut citer le propylène glycol.

Parmi les agents mouillants qui ont pour rôle de diminuer la tension superficielle et de permettre un plus grand étalement du liquide, on utilise préférentiellement, sans que cette liste soit limitative, des composés tels que les Pluronics type L44NF vendus par BASF ; les synperonics type PE/L62 et PE/L44 vendus par Uniquema.

Parmi les agents conservateurs, on peut citer à titre d'exemples non limitatifs l'acide benzoïque et ses dérivés avec l'alcool benzylique, le chlorure de benzalkonium, le benzoate de sodium, le bronopol, la chlorhexidine, le chlorocrésol et ses dérivés, l'alcool éthylique, l'alcool phénéthylique, le phénoxyéthanol, le sorbate de potassium, la diazolidinylurée, les parabènes tels que le propyl parabène ou le méthyl parabène, pris seuls ou en mélanges.

A titre d'agent conservateur préféré, on peut citer les parabènes, le phénoxyéthanol ou le chlorure de benzalkonium, pris seuls ou en mélange.

La composition selon l'invention peut comprendre un ou plusieurs émulsionnants.

Les émulsionnants tensio-actifs sont des composés amphiphiles qui possèdent une partie hydrophobe ayant une affinité pour l'huile et une partie hydrophile ayant une affinité pour l'eau créant ainsi un lien entre les deux phases. Les émulsionnants ioniques ou non ioniques stabilisent donc les émulsions huile/eau en s'adsorbant à l'interface et en formant des couches lamellaires de cristaux liquides.

Le pouvoir émulsionnant des tensio-actifs non-ioniques est étroitement lié à la polarité de la molécule. Cette polarité est définie par le HLB (Balance Hydrophile/Lipophile).

Une HLB élevée indique que la fraction hydrophile est prédominante, et, à l'inverse, une faible HLB indique que la partie lipophile est prédominante. Par exemple, des valeurs de HLB supérieures à environ 10 correspondent à des tensioactifs hydrophiles.

Les tensioactifs peuvent être classés, selon leur structure, sous les termes génériques "ioniques" (anioniques, cationiques, amphotères) ou "non ioniques". Les tensioactifs non ioniques sont des tensioactifs qui ne se dissocient pas en ions dans l'eau et sont donc insensibles aux variations de pH.

De préférence, on utilisera, en tant que système émulsionnant un ou plusieurs couples "tensioactif non ionique de haute HLB" / "tensioactif non ionique de faible HLB", il pourra en particulier s'agir d'un système émulsionnant non ionique comprenant au moins un tensioactif non ionique présentant une HLB supérieure à environ 10 et au moins un tensioactif non ionique présentant une HLB inférieure à environ 10.

Le ratio de chacun des deux tensioactifs formant le couple précité est déterminé le plus souvent par le calcul de la HLB requise de la phase grasse utilisée.

A titre d'émulsifiants préférés on peut citer des émulsifiants hydrophiles de type Tween 80, Glyceryl Manostearate & POE Stearate vendu sous le nom Arlacel 165FL® par la société Uniquema, les copolyéthers vendus sous le nom de Synperonics type PE/F68 par Uniquema; des émulsifiants lipophiles de type Glucate SS et Glucamate SSE, Polyoxyethylene (21) Stearyl Ether vendu sous le nom Brij721® par la société Uniquema. On peut citer également des tensioactifs non ioniques présentant une HLB élevée, les esters de sorbitan tels que le POE(20) sorbitan monooléate, vendu sous le nom de « Tween 80 » (HLB=15) ; le POE(20) sorbitan monostéarate vendu sous le nom de « Tween 60 » (HLB=14.9) ; les éthers d'alcools gras tels que le POE (21) stéaryl ether (HLB= 15.5), ou le ceteareth 20 vendu sous le nom de « Eumulgin B2 » par Cognis (HLB de 15,5, ou des tensioactifs non ioniques de basse HLB (lipophiles), les esters de sorbitan, tels que le monostéarate de sorbitan (vendu sous le nom de Span 60 par Unichema), les esters de glycérol (vendu sous le nom de Cutina GMSVPH par Cognis) tels que le monostéarate de glycérol (Cutina GMS de chez Cognis), les esters de saccharose de bas HLB comme le distéarate de saccharose.

La composition selon l'invention peut également comprendre une phase grasse. Cette phase grasse peut comprendre par exemple, les huiles végétales, minérales, animales ou synthétiques, des huiles de silicones, et leurs mélanges.

Comme exemple d'huile minérale, on peut citer par exemple des huiles de paraffine de différentes viscosités telles que le Primol 352®, le Marcol 82®, Marcol 152® vendus par la société Esso.

Comme huile végétale, on peut citer l'huile d'amande douce, l'huile de palme, l'huile de soja, l'huile de sésame, l'huile de tournesol.

Comme huile animale, on peut citer la lanoline, le squalene, l'huile de poisson, l'huile de vison avec comme dérivé le squalane vendu sous le nom Cosbiol® par la société Laserson.

Comme huile synthétique, on peut citer un ester tel que le cetearyl isononanoate comme le produit vendu sous le nom de Cetiol SN® par la société Cognis France, le diisopropyl adipate comme le produit vendu sous le nom de Ceraphyl 230® par la société ISF, le palmitate d'isopropyle comme le produit vendu sous le nom de Crodamol IPP® par la société Croda, le caprylique caprique triglycéride tel que Miglyol 812® vendu par la société Huls / Lambert Rivière.

Comme huile de silicone, on peut citer une dimethicone comme le produit vendu sous le nom de Dow Corning 200 fluid®, une cyclomethicone comme le produit vendu sous le nom de Dow Corning 244 fluid® par la société Dow Corning ou le produit vendu sous le nom le Mirasil CM5® par la société SACI-CFPA.

On pourra également mettre des corps gras solides tel que des cires naturelles ou synthétiques. Dans ce cas, l'homme du métier adaptera la température de chauffage de la préparation en fonction de la présence ou non de ces solides.

Pour la composition selon l'invention, les huiles de paraffine et plus particulièrement le Marcol 152® et le Miglyol 812® sont préférées.

Les compositions de l'invention peuvent comprendre en outre tout additif usuellement utilisé dans le domaine cosmétique ou pharmaceutique tel que des tensioactifs, des neutralisants, des filtres solaires, des antioxydants, des charges, des électrolytes, des colorants, des bases ou acides usuels, minéraux ou organiques, des parfums, des huiles essentielles, des actifs cosmétiques, des hydratants, des vitamines, des acides gras essentiels, des sphingolipides, des composés autobronzants tels que la DHA, des agents apaisants et protecteurs de la peau tels que l'allantoïne, des agents propénétrants, ou un mélange de ceux-ci. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées.

Ces additifs peuvent être présents dans la composition à raison de 0,001% à 20 % en poids par rapport au poids total de la composition.

Dans un mode particulier de réalisation de l'invention, la composition se présente sous forme d'une émulsion huile-dans-eau (H/E) de type lotion, crème ou gel-crème et comprend :
- de 0,1% à 0,3% d'un dérivé de l'acide naphtoïque ;
- de 1% à 10% d'au moins un agent filmogène ;
- de 0,1% à 3% d'agents gélifiants ;
- de 0,01% à 1,5% d'agents chélatants ;
- de 0,1% à 10% d'un agent mouillant ;
- de 0,1% à 20% d'un agent émollient ;
- de 0,1% à 30% de phase grasse ;
- de 0,01% à 3% d'agents conservateurs ;
- de 0 à 10% d'émulsifiants.

Dans un mode particulièrement préféré, la composition se présente sous forme gel et comprend :
- de 0,1% à 0,3% d'un dérivé de l'acide naphtoïque ;
- de 1% à 10% d'au moins un agent filmogène;
- de 0,1% à 3% d'agents gélifiants ;
- de 0,01% à 1,5% d'agents chélatants ;
- de 0,1% à 10% d'un agent mouillant ;
- de 0,01% à 3% d'agents conservateurs.

Optionnellement, la composition gel comprend de plus :
- de 0,1% à 20% d'un agent émollient.

La présente invention a aussi pour objet la composition telle que décrite précédemment à titre de médicament. La composition peut en effet être utilisée comme médicament.

L'invention a également pour objet un procédé de préparation d'une composition telle que décrite précédemment. Un tel procédé est caractérisé en ce qu'il comprend l'étape de mélange d'un véhicule physiologiquement acceptable comprenant au moins un dérivé de l'acide naphtoïque avec au moins un agent filmogène choisi parmi les composés selon la revendication 1, afin d'obtenir une composition dans laquelle ledit dérivé d'acide naphtoïque de formule (I) telle que définie dans la revendication est sous une forme dispersée.

L'introduction des autres excipients et additifs éventuels se fera en fonction de la nature chimique des composés et de la forme galénique choisie.

La préparation d'une composition selon l'invention se fait en 3 ou 5 étapes selon la forme galénique choisie, les 2 étapes supplémentaires étant effectuées uniquement pour la préparation des formes de types émulsions telle que crèmes, lotions, gels-crèmes.

L'agent filmogène étant de nature hydrophile, il est introduit lors de la préparation de la phase hydrophile lorsqu'il s'agit d'une émulsion. Dans le cas d'un gel, l'agent filmogène est introduit dans la phase aqueuse après dispersion du ou des gélifiants et avant l'étape de neutralisation, en fonction de la nature du ou des agents gélifiants.

Ainsi, le procédé selon l'invention comprend les étapes suivantes:
a) mélange du dérivé d'acide naphtoïque avec optionnellement au moins un agent mouillant et/ou au moins un agent chélatant et/ou au moins un gélifiant et/ou des émulsifiants hydrophiles et/ou un ou plusieurs émollients (de préférence au moins un agent mouillant et au moins un agent chélatant), dans de l'eau, jusqu'à ce que ledit dérivé d'acide naphtoïque soit parfaitement dispersé, afin d'obtenir la phase active aqueuse ;
b) mélange d'au moins un agent filmogène avec de l'eau afin d'obtenir une phase filmogène ;
c) introduction de la phase filmogène obtenue en b) dans la phase active aqueuse obtenue en a) ou inversement, afin d'obtenir une composition aqueuse.

En fonction de la nature du gélifiant, celui-ci pourra être introduit directement au sein de la phase active aqueuse ou préparé dans une phase gélifiante indépendante et ajoutée aux autres phases constituant la composition selon l'invention.

Lorsque la composition selon l'invention est une émulsion comprenant une phase grasse de type crèmes, lotions ou gels-crèmes, le procédé comprend également, à l'issue de l'étape c), une étape de mélange de la composition aqueuse obtenue en c) avec au moins une phase grasse afin d'obtenir une émulsion.
Ladite phase grasse peut être obtenue par mélange d'au moins un émulsifiant lipophile, avec au moins une huile et/ou un corps gras solide.

L'invention se rapporte également à l'utilisation de la nouvelle composition telle que décrite précédemment en cosmétique et en dermatologie.

En particulier, l'invention se rapporte à l'utilisation d'une composition telle que décrite précédemment pour la préparation d'une composition pharmaceutique destinée au traitement et/ou à la prévention des affections dermatologiques liées à un désordre de la kératinisation portant sur la différenciation et sur la prolifération cellulaire notamment pour traiter les acnés vulgaires, comédoniennes, papulopustuleuse, papulocomédoniennes, nodulokystiques, les acnés conglobata, les acnés chéloïdes de la nuque, les acnés miliaires récidivantes, les acnés nécrotiques, les acnés neonatorum, les acnés professionnelles, les acnés rosacées, les acnés séniles, les acnés solaires et les acnés médicamenteuses. Particulièrement, l'invention a pour objet la composition selon l'invention pour son utilisation dans le traitement et/ou la prévention des affections dermatologiques liées à un désordre de la kératinisation portant sur la différenciation et sur la prolifération cellulaire, notamment pour traiter les acnés vulgaires, comédoniennes, papulopustuleuse, papulocomédoniennes, nodulokystiques, les acnés conglobata, les acnés chéloïdes de la nuque, les acnés miliaires récidivantes, les acnés nécrotiques, les acnés neonatorum, les acnés professionnelles, les acnés rosacées, les acnés séniles, les acnés solaires et les acnés médicamenteuses.

Plus particulièrement, l'invention se rapporte à l'utilisation d'une composition telle que décrite précédemment pour la préparation d'une composition pharmaceutique destinée à prévenir et/ou à traiter les acnés vulgaires.

Préférentiellement, lesdites compositions selon l'invention sont administrées par voie topique. Par voie topique, on entend une administration sur la peau, les phanères ou les muqueuses.

En outre, l'invention porte également sur l'utilisation cosmétique d'une composition selon l'invention pour lutter contre l'aspect gras de la peau ou des cheveux, dans le traitement des peaux physiologiquement grasses, ou pour prévenir et/ou lutter contre le vieillissement photo-induit ou chronologique.

La présente invention va maintenant être illustrée au moyen des exemples suivants :

### Exemple 1 : Exemple d'un procédé de préparation d'une composition selon l'invention

### Etape a: préparation de la phase active aqueuse:

Dans un bêcher qui servira de récepteur du produit fini, on introduit sous agitation avec une défloculeuse de l'eau purifiée, le principe actif (adapalène), optionnellement les émulsifiants hydrophiles (type Arlacel 165FL, Tween 80) dans le cas de la réalisation d'une émulsion huile dans eau, les émollients (type glycérine), les agents mouillants (type Synperonic PE/L62, Synperonic PE/L44), l'agent chélatant (type EDTA), optionnellement à ce stade le ou les gélifiants (type Carbopol, Pemulen TR1, Xantural, Methocel, Simulgel 600). On laisse sous agitation à froid jusqu'à parfaite dispersion. Lorsque le mélange est homogène, la phase aqueuse est portée à 60 °C au Bain Marie et on introduit le conservateur (type méthyl parabène).

### Etape b : Préparation de la phase filmogène :

- Au moins un agent filmogène selon la revendication 1 est mélangé à de l'eau purifiée.

### Etape c : Introduction de la phase filmogène obtenue en b) dans la phase active aqueuse obtenue en a) ou inversement.

### Etape d (optionnelle): Préparation de la phase grasse :

Dans un bêcher annexe, on introduit sous agitation avec une défloculeuse les émulsifiants lipophiles type (Glucate SS, Glucamate SSE 20, Brij 721), les composés huileux (type olépal isostéarique, Cetiol SN, Crodamol DA, Speziol C18, Miglyol 812, Cosbiol) et les conservateurs (type phénoxyéthanol et propyl parabène). Le mélange est porté à 60 °C au bain Marie et après homogénéisation, on introduit la silicone volatile si cette dernière est présente dans la composition.

### Etape e (optionnelle): Emulsification :

A la température de 60°C et sous agitation avec une défloculeuse, la phase grasse est introduite doucement dans la phase aqueuse afin de réaliser l'émulsification. Le chauffage est maintenu 5 minutes, puis la plaque chauffante est retirée pour laisser le produit refroidir doucement. L'agitation est réglée en fonction de la viscosité.

Les étapes d et e ci-dessus sont optionnelles et sont effectuées uniquement pour la préparation des formes de types émulsions telle que crèmes, lotions, ou gel-crèmes.

### Etape f : Neutralisation :

A 40°C, l'agent de neutralisation du gélifiant (type triéthanoloamine ou solution d'hydroxyde de sodium à 10%) est introduit si nécessaire, jusqu'à un pH de 5,5 +/- 0,5. Le produit prend alors une consistance plus épaisse. A la fin de la fabrication, le pH est à nouveau vérifié. S'il est dans les normes, le qsp en eau est effectué. Le produit est homogénéisé une dernière fois afin de s'assurer de la bonne dispersion du principe actif Adapalène (observation microscopique révélant une dispersion homogène et sans agrégats), puis le produit est conditionné.

### Exemple 2 : Formulations de type gel contenant de l'adapalène à 0,1% avec du Kollidon VA64 (copovidone) en tant qu'agent filmogène

| Ingrédients | Formule A | Formule B |
|---|---|---|
| Adapalène | 0.10% | 0.10% |
| Eau purifiée | 79.50% | 79.50% |
| Carbopol 980 NF | 0.75% | 0.75% |
| Méthyl parabène | 0.10% | 0.10% |
| EDTA | 0.10% | 0.10% |
| Propylène glycol | 4.00% | 4.00% |
| PEG 400 | 4.00% | 4.00% |
| Kollidon VA64 | 4.00% | 6.00% |
| Pluronic L44NF | 0.20% | 0.20% |
| Solution sodium hydroxyde (10% m/m) | Qsp pH 5.5±0.5 | Qsp pH 5.5±0.5 |
| Eau purifiée | Qsp 100% | Qsp 100% |

### Procédé de fabrication des formulations A et B :

1) Dans un bécher, peser une partie de l'eau purifiée et le Carbopol 980 NF et mélanger jusqu'à dispersion totale à 800 tr/min
2) Ajouter le méthyl parabène, l'EDTA et mélanger jusqu'à dissolution totale à 800 tr/min
3) Dans un autre bécher, peser l'autre partie de l'eau purifiée et introduire le propylène glycol, le PEG 400, le Kollidon VA64 sous agitation à 800 tr/min
4) Après dissolution totale, ajouter l'adapalène, le PLuronic L44NF et mélanger à 500 tr/min
5) Introduire la phase active dans le premier bécher et mélanger à 600 tr/min
6) Neutraliser avec la solution d'hydroxyde de sodium jusqu'à pH 5.5±0.5
7) Ajuster à 100% avec l'eau purifiée si nécessaire

### Exemple 3 : Formulations de type ciel contenant de l'adapalène à 0,1% avec du Hyaluronate de sodium en tant qu'agent filmogène

| Ingrédients | Formule C |
|---|---|
| Adapalène | 0.10% |
| Eau purifiée | 77.50% |
| Hyaluronate de sodium | 2.00% |
| Phénoxyéthanol | 1.00% |
| EDTA | 0.20% |
| Propylène glycol | 4.00% |
| Synperonic PE/L62 | 0.20% |
| Eau purifiée | Qsp 100% |

### Procédé de fabrication de la formulation C :

1) Dans un bécher, peser une partie de l'eau purifiée et le hyaluronate de sodium et mélanger à 800 tr/min
2) Dans une autre bécher, peser l'autre partie de l'eau, l'EDTA, le propylène glycol, le synperonic PE/L62, le phénoxyéthanol et mélanger jusqu'à l'obtention d'une solution limpide à 800 tr/min
3) Introduire l'adapalène et disperser à l'Ultra-turrax à 2400 tr/min pendant 3 minutes
4) Introduire la phase active ainsi préparée dans le premier bécher et homogénéiser à 800 tr/min
5) Ajuster à 100% avec l'eau purifiée si nécessaire

### Exemple 4 : Etude de stabilité des compositions selon l'invention

### a) Gel Kollidon VA64

| Temps | Conditions | Formule A | | Formule B | |
|---|---|---|---|---|---|
| | | pH | Dosage (%) | pH | Dosage (%) |
| T zéro | NA | 5.1 | 97.1 | 5.1 | 97.1 |
| 1 mois | 5 °C | 5.1 | 102.3 | 5.1 | 96.2 |
| | 25 °C/60HR | 5.1 | 95.1 | 5.1 | 92.9 |
| | 40 °C/75HR | 5.1 | 96.7 | 4.9 | 97.7 |
| 2 mois | 5 °C | 5.1 | 96.0 | 5.0 | 98.4 |
| | 25 °C/60HR | 5.1 | 97.0 | 5.1 | 101.9 |
| | 40 °C/75HR | 5.1 | 97.6 | 4.9 | 96.7 |
| 3 mois | 5°C | 5.0 | 95.5 | 4.9 | 97.0 |
| | 25 °C/60HR | 5.0 | 96.1 | 5.0 | 97.4 |
| | 40 °C/75HR | 4.9 | 97.7 | 4.7 | 97.7 |

### b) Gel Hyaluronate de sodium

| Temps | Conditions | Formule C |
|---|---|---|
| | | Dosage(%) |
| T zéro | NA | 99.5 |
| 1 mois | Température ambiante | 97.9 |
| | 40 °C/75HR | |
| 3 mois | Température ambiante | 100.1 |
| | 40 °C/75HR | 100.2 |

### Exemple 5 : Etude de Tolérance des gels avec agents filmogènes contenant 0.1% d'adaplène vs Différine Gel 0.1%

La présente étude a pour but de comparer le pouvoir irritant d'un gel de référence à 0,1% d'adapalène avec celui de 3 formulations d'adapalène 0,1% sous forme de gel contenant un agent filmogène à différentes concentrations, ainsi que leurs placebos, sur la peau de l'oreille de la souris BALB/c après applications topiques répétées pendant 6 jours.

L'application topique quotidienne (20 µl) des produits à tester est réalisée sur la face interne de l'oreille de souris BALB/c réparties en dix groupes (souris femelles et âgées de 8 semaines environ) à raison d'une application par jour pendant 6 jours.

Les formulations testées sont les suivantes :
- Non traité
- Différine Gel 0.1% (DG)
- Différine Gel placebo (DG plac.)
- Gel Kollidon VA64 placebo (AB plac.)
- Gel Kollidon VA64 (4%) 0.1% (Formule A)
- Gel Kollidon VA64 (6%) 0.1%(Formule B)
- Gel Hyaluronate placebo(C plac.)
- Gel Hyaluronate 0.1% (Formule C)

| | ASC* J2-J19 | | % augmentation ASC | T-test Student |
|---|---|---|---|---|
| | Moyenne | Ecart-type | Vs. non traité | Vs. non traité |
| Non traité | 352,0 | 6,3 | -- | -- |
| Différine Gel placebo | 352,0 | 1,1 | 0,0 | NS |
| Différine Gel 0.1% | 519,8 | 43,1 | 47,7 | ** |
| Gel Kollidon VA64 placebo | 359,8 | 3,9 | 2,2 | NS |
| Formule A : Gel Kollidon VA64 (4%) 0.1% | 403,9 | 18,6 | 14,7 | * |
| Formule B : Gel Kollidon VA64 (6%) 0.1% | 404,5 | 32,8 | 14,9 | NS |
| Gel Hyaluronate placebo | 361,1 | 2,7 | 2,6 | NS |
| Gel Hyaluronate 0.1% Formule C | 433,5 | 18,4 | 23,2 | ** |

| | | | | |
|---|---|---|---|---|
| *: Aire sous la courbe | | | | |

Les formulations filmogènes placebo Kollidon VA64 et Hyaluronate de sodium ne sont pas irritantes.

Les formulations filmogènes Kollidon VA64 et Hyaluronate de sodium sont moins irritantes que la référence commerciale Différine Gel.

Différine Gel 0.1% augmente l'aire sous la courbe de 48% par rapport au groupe non traité.

Les gels Kollidon VA64 0.1%, quelle que soit la teneur en Kollidon VA64 (4 ou 6%), augmentent l'aire sous la courbe de 15% par rapport au groupe non traité.

Le gel Hyaluronate de sodium 0.1% augmente l'aire sous la courbe de 23% par rapport au groupe non traité.

Les formulations filmogènes Kollidon VA64 et Hyaluronate de sodium avec 0.1% d'adapalène sont moins irritantes que Différine Gel 0.1%.

En effet, la comparaison des aires sous la courbe montre que :
- les gels Kollidon VA64 4% et 6%, avec 0.1% d'adapalène, diminuent l'aire sous la courbe de 22% versus Différine Gel 0.1% ;
- le gel Hyaluronate de sodium avec 0.1% d'adapalène diminue l'aire sous la courbe de 16% versus Différine Gel 0.1%.

### Exemple 6 : Etude de libération-pénétration in vitro du gel Kollidon VA64

### (4%) (Formule A) contenant 0.1% d'adapalène vs Différine Gel 0.1% (DG)

La présente étude a pour but de comparer in vitro la libération-pénétration à travers la peau humaine sans occlusion de l'adapalène formulé à 0,1% (m/m) dans un gel contenant 4% de Kollidon VA 24 (Formule A) et d'un gel de référence à 0.1% d'adapalène.

Les études d'absorption ont été conduites en utilisant une peau humaine excisée montée en conditions statiques pendant une période de 16 heures. Trois échantillons de peau ont été utilisés. Une quantité de 10 mg de chaque formule (10µg d'adapalène) a été appliquée sur une surface de 1 cm² de peau. Les concentrations d'adapalène dans les fractions fluides recueillies au cours du temps et restant dans la peau à la fin de l'étude ont été évaluées par la méthode HPLC avec détection de la fluorescence (fondé sur une méthode validée. Limite de quantification : 1ng.mL⁻¹).

| | Différine Gel 0.1% | Formule A (Filmogène Kollidon VA64 (4%)) |
|---|---|---|
| Concentration (% m/m) | 0.1 | 0.1 |
| Dose déposée (µg) | 7.03±1.41 | 5.69±0.45 |
| N | 6 | 6 |
| SC+Epidermis | | |
| ng | 49.63±35.72 | 60.94±17.12 |
| % dose | 0.75±0.59 | 1.08±0.30 |
| Dermis | | |
| ng | 4.12±5.12 | 1.37±1.46 |
| % dose | 0.01±0.01 | 0.03±0.03 |
| Dose absorbée | | |
| ng | 0.62±0.95 | ILQ (Inférieur à la Limite de Quantification) ILQ |
| % dose | 0.01±0.01 | |
| Quantité totale pénétrée | | |
| ng | 54.36±32.10 | 62.31±17.77 |
| % dose | 0.82±0.54 | 1.10±0.32 |
| Masse balance | | |
| µg | 6.05±1.15 | 4.95±0.56 |
| % dose | 86.21±3.14 | 86.87±4.62 |

### Conclusions de l'étude :

- Les formulations Différine Gel 0.1% et Gel Kollidon VA64 présentent le même profil de distribution SC+Epiderme / Derme avec la majeure partie de l'adapalène présent dans les couches supérieures cutanées.
- La quantité totale pénétrée d'adapalène est légèrement supérieure avec la formulation Gel Kollidon VA64.

### Conclusion générale

La formulation gel contenant du Kollidon VA64 améliore la tolérance de l'adapalène sans modifier le profil de distribution ni la quantité totale pénétrée.

## Revendications

1. Composition comprenant, dans un milieu physiologiquement acceptable, au moins un dérivé de l'acide naphtoïque et au moins un agent filmogène, **caractérisée en ce que** le dérivé d'acide naphtoïque est sous une forme dispersée dans ladite composition et est un composé de formule (I) : 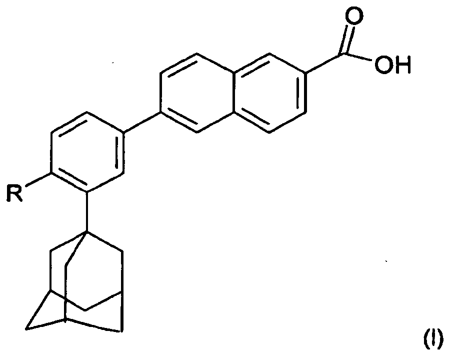 où R représente un atome d'hydrogène, un radical hydroxyle, un radical alkyle ramifié ou non, ayant de 1 à 4 atomes de carbone, un radical alkoxy ayant de 1 à 10 atomes de carbone ou un radical cycloaliphatique,
et **en ce que** l'agent filmogène est choisi parmi les polyvinylpyrrolidones, les dérivés acétates de vinyle, les alcools polyvinyliques, les polymères cyanoacryliques, les copolymères acryliques/méthacryliques et polyméthacrylate/butylacrylate, et le hyaluronate de sodium.

2. Composition selon la revendication 1, **caractérisée en ce que** la concentration du dérivé de l'acide naphtoïque est comprise entre 0,001% et 10%, préférentiellement entre 0,01% et 5% et, plus préférentiellement, entre 0,05% et 2% en poids du poids total de la composition.

3. Composition selon l'une des revendications précédentes, **caractérisée en ce que** le dérivé d'acide naphtoïque est choisi parmi l'acide 6-[3-(1-adamantyl)-4-methoxyphenyl]-2-naphtoïque, l'acide 6-[3-(1-adamantyl)-4-hydroxyphenyl]-2-naphtoïque, l'acide 6-[3-(1-adamantyl)-4-decyloxyphenyl]-2-naphtoïque et l'acide 6-[3-(1-adamantyl)-4-hexyloxyphenyl]-2-naphtoïque.

4. Composition selon l'une des revendications 1 à 3, **caractérisée en ce que** le dérivé d'acide naphtoïque est l'acide 6-[3-(1-adamantyl)-4-méthoxyphényl]-2-naphtoïque.

5. Composition selon la revendication 4, **caractérisée en ce que** la concentration en acide 6-[3-(1-adamantyl)-4-méthoxyphényl]-2-naphtoïque est d'environ 0,1% en poids par rapport au poids total de la composition.

6. Composition selon la revendication 4, **caractérisée en ce que** la concentration en acide 6-[3-(1-adamantyl)-4-méthoxyphényl]-2-naphtoïque est d'environ 0,3% en poids par rapport au poids total de la composition.

7. Composition selon l'une quelconque des revendications précédentes, **caractérisée en ce que** l'agent filmogène est choisi parmi le hyaluronate de sodium et la copovidone.

8. Composition selon l'une quelconque des revendications précédentes, **caractérisée en ce que** la concentration en agent filmogène est comprise entre 0,5% et 20% en poids par rapport au poids total de la composition, préférentiellement, entre 1% et 10%.

9. Composition selon la revendication 8, **caractérisée en ce que** la concentration en agent filmogène est de 2%, 4%, ou 6% en poids du poids total de la composition.

10. Composition selon l'une quelconque des revendications précédentes, **caractérisée en ce qu'**elle se présente sous la forme d'une dispersion aqueuse, hydroalcoolique ou huileuse, d'un gel aqueux, anhydre ou lipophile, d'une émulsion de consistance liquide, semi-liquide ou solide obtenue par dispersion d'une phase grasse dans une phase aqueuse ou inversement, ou d'une suspension de consistance molle, semi-liquide ou solide ou encore d'une micro émulsion, de micro capsules, de micro particules ou de dispersions vésiculaires de type ionique et/ou non ionique.

11. Composition selon la revendication 10, **caractérisée en ce qu'**elle se présente sous la forme d'un gel.

12. Composition selon la revendication 10, **caractérisée en ce qu'**elle se présente sous la forme d'une crème.

13. Composition selon la revendication 10, **caractérisée en ce qu'**elle se présente sous la forme d'une lotion.

14. Composition selon la revendication 10, **caractérisée en ce qu'**elle se présente sous la forme d'un gel-crème.

15. Composition selon la revendication 11, **caractérisée en ce qu'**elle comprend dans l'eau :
- de 0,1% à 0,3% d'un dérivé de l'acide naphtoïque ;
- de 1% à 10% d'au moins un agent filmogène;
- de 0,1% à 3% d'agents gélifiants ;
- de 0,01% à 1,5% d'agents chélatants ;
- de 0,1% à 10% d'un agent mouillant ; et
- de 0,01 % à 3% d'agents conservateurs.

16. Composition selon la revendication 11, **caractérisée en ce qu'**elle comprend dans l'eau :
- de 0,1% à 0,3% d'un dérivé de l'acide naphtoïque ;
- de 1% à 10% d'au moins un agent filmogène;
- de 0,1 % à 3% d'agents gélifiants ;
- de 0,01% à 1,5% d'agents chélatants ;
- de 0,1% à 10% d'un agent mouillant ; et
- de 0,1% à 20% d'un agent émollient ;
- de 0,01% à 3% d'agents conservateurs.

17. Composition selon l'une des revendications 12 à 14, **caractérisée en ce qu'**elle comprend dans l'eau:
- de 0,1% à 0,3% d'un dérivé de l'acide naphtoïque ;
- de 1 % à 10% d'au moins un agent filmogène;
- de 0,1 % à 3% d'agents gélifiants ;
- de 0,01% à 1,5% d'agents chélatants ;
- de 0,1% à 10% d'un agent mouillant ;
- de 0,1% à 20% d'un agent émollient ;
- de 0,1% à 30% de phase grasse ;
- de 0,01% à 3% d'agents conservateurs ;
- de 0 à 10% d'émulsifiants.

18. Composition selon l'une quelconque des revendications précédentes pour une utilisation à titre de médicament.

19. Procédé de préparation d'une composition selon l'une quelconque des revendications 1 à 17, **caractérisé en ce qu'**il comprend l'étape de mélange d'un véhicule physiologiquement acceptable comprenant au moins un dérivé de l'acide naphtoïque avec au moins un agent filmogène, afin d'obtenir une composition dans laquelle ledit dérivé d'acide naphtoïque est sous une forme dispersée dans ladite composition.

20. Procédé de préparation selon la revendication 19, **caractérisé en ce qu'**il comprend les étapes suivantes:
a) mélange du dérivé d'acide naphtoïque avec au moins un agent mouillant, et au moins un agent chélatant dans de l'eau, jusqu'à ce que ledit dérivé d'acide naphtoïque soit parfaitement dispersé, afin d'obtenir la phase active aqueuse ;
b) mélange d'au moins un agent filmogène avec de l'eau afin d'obtenir une phase filmogène ;
c) introduction de la phase filmogène obtenue en b) dans la phase active aqueuse obtenue en a) ou inversement, afin d'obtenir une composition aqueuse.

21. Procédé de préparation selon la revendication 20, **caractérisé en ce qu'**il comprend en outre, à l'issue de l'étape c), une étape de mélange de la composition aqueuse obtenue en c) avec au moins une phase grasse afin d'obtenir une émulsion.

22. Utilisation d'une composition selon l'une des revendications 1 à 17 pour la préparation d'une composition pharmaceutique destinée au traitement et/ou à la prévention des affections dermatologiques liées à un désordre de la kératinisation portant sur la différenciation et sur la prolifération cellulaire choisies parmi les acnés vulgaires, comédoniennes, papulopustuleuse, papulocomédoniennes, nodulokystiques, les acnés conglobata, les acnés chéloïdes de la nuque, les acnés miliaires récidivantes, les acnés nécrotiques, les acnés neonatorum, les acnés professionnelles, les acnés rosacées, les acnés séniles, les acnés solaires et les acnés médicamenteuses.

23. Utilisation d'une composition selon la revendication 22 pour la préparation d'une composition pharmaceutique destinée à prévenir et/ou traiter les acnés vulgaires.

24. Utilisation cosmétique d'une composition selon l'une quelconque des revendications 1 à 17 pour lutter contre l'aspect gras de la peau ou des cheveux, dans le traitement des peaux physiologiquement grasses ou pour prévenir et/ou lutter contre le vieillissement photo-induit ou chronologique.

## Patentansprüche

1. Zusammensetzung umfassend in einem physiologisch verträglichen Milieu wenigstens ein Naphthoesäurederivat und wenigstens ein filmbildendes Mittel, **dadurch gekennzeichnet, dass** das Naphthoesäurederivat in dispergierter Form in besagter Zusammensetzung vorliegt und eine Verbindung der Formel (I) ist: 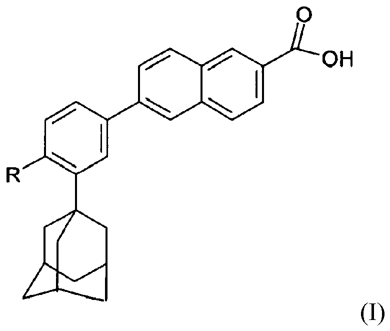 wo R für ein Wasserstoffatom, einen Hydroxylrest, einen linearen oder verzweigten Alkylrest mit 1 bis 4 Kohlenstoffatomen, einen Alkoxyrest mit 1 bis 10 Kohlenstoffatomen oder einen cycloaliphatischen Rest steht,
und dass das filmbildende Mittel aus Polyvinylpyrrolidonen, Vinylacetatderivaten, Polyvinylalkoholen, Cyanoacrylpolymeren, Acryl/Methacryl- und Polymethacrylat/Butylacrylat-Copolymeren und Natriumhyaluronat ausgewählt ist.

2. Zusammensetzung gemäß Anspruch 1, **dadurch gekennzeichnet, dass** die Konzentration des Naphthoesäurederivates zwischen 0,001 Gew.-% und 10 Gew.-%, bevorzugt zwischen 0,01 Gew.-% und 5 Gew.-%, bevorzugter zwischen 0,05 Gew.-% und 2 Gew.-% des Gesamtgewichtes der Zusammensetzung beträgt.

3. Zusammensetzung gemäß einem der vorhergehenden Ansprüche, **dadurch gekennzeichnet, dass** das Naphthoesäurederivat aus 6-[3-(1-Adamantyl)-4-methoxyphenyl]-2-naphthoesäure, 6-[3-(1-Adamantyl)-4-hydroxyphenyl]-2-naphthoesäure, 6-[3-(1-Adamantyl)-4-decyloxyphenyl]-2-naphthoesäure und 6-[3-(1-Adamantyl)-4-hexyloxyphenyl]-2-naphthoesäure ausgewählt ist.

4. Zusammensetzung gemäß einem der Ansprüche 1 bis 3, **dadurch gekennzeichnet, dass** das Naphthoesäurederivat 6-[3-(1-Adamantyl)-4-methoxyphenyl]-2-naphthoesäure ist.

5. Zusammensetzung gemäß Anspruch 4, **dadurch gekennzeichnet, dass** die Konzentration der 6-[3-(1-Adamantyl)-4-methoxyphenyl]-2-naphthoesäure ungefähr 0,1 Gew.-% bezogen auf das Gesamtgewicht der Zusammensetzung beträgt.

6. Zusammensetzung gemäß Anspruch 4, **dadurch gekennzeichnet, dass** die Konzentration der 6-[3-(1-Adamantyl)-4-methoxyphenyl]-2-naphthoesäure ungefähr 0,3 Gew.-% bezogen auf das Gesamtgewicht der Zusammensetzung beträgt.

7. Zusammensetzung gemäß einem der vorhergehenden Ansprüche, **dadurch gekennzeichnet, dass** das filmbildende Mittel aus Natriumhyaluronat und Copovidon ausgewählt ist.

8. Zusammensetzung gemäß einem der vorhergehenden Ansprüche, **dadurch gekennzeichnet, dass** die Konzentration des filmbildenden Mittels zwischen 0,5 Gew.-% und 20 Gew.-%, bevorzugt zwischen 1 Gew.-% und 10 Gew.-%, bezogen auf das Gesamtgewicht der Zusammensetzung beträgt.

9. Zusammensetzung gemäß Anspruch 8, **dadurch gekennzeichnet, dass** die Konzentration des filmbildenden Mittels 2 Gew.-%, 4 Gew.-% oder 6 Gew.-% des Gesamtgewichtes der Zusammensetzung beträgt.

10. Zusammensetzung gemäß einem der vorhergehenden Ansprüche, **dadurch gekennzeichnet, dass** sie als wässrige, hydroalkoholische oder ölige Dispersion, als wässriges, wasserfreies oder lipophiles Gel, als Emulsion mit flüssiger, halbflüssiger oder fester Konsistenz, die durch Dispersion einer Fettphase in einer Wasserphase oder umgekehrt erhalten wird, oder als eine Suspension mit weicher, halbflüssiger oder fester Konsistenz oder auch als eine Mikroemulsion, Mikrokapseln, Mikropartikel oder Dispersionen ionischer und/oder nichtionischer Vesikel vorliegt.

11. Zusammensetzung gemäß Anspruch 10, **dadurch gekennzeichnet, dass** sie als Gel vorliegt.

12. Zusammensetzung gemäß Anspruch 10, **dadurch gekennzeichnet, dass** sie als Creme vorliegt.

13. Zusammensetzung gemäß Anspruch 10, **dadurch gekennzeichnet, dass** sie als Lotion vorliegt.

14. Zusammensetzung gemäß Anspruch 10, **dadurch gekennzeichnet, dass** sie als Gel-Creme vorliegt.

15. Zusammensetzung gemäß Anspruch 11, **dadurch gekennzeichnet, dass** sie in Wasser umfasst:
- 0,1% bis 0,3% eines Naphthoesäurederivates;
- 1% bis 10% wenigstens eines filmbildenden Mittels;
- 0,1 % bis 3% Gelbildner;
- 0,01% bis 1,5% Chelatbildner;
- 0,1% bis 10% eines Benetzungsmittels; und
- 0,01% bis 3% Konservierungsmittel.

16. Zusammensetzung gemäß Anspruch 11, **dadurch gekennzeichnet, dass** sie in Wasser umfasst:
- 0,1% bis 0,3% eines Naphthoesäurederivates;
- 1% bis 10% wenigstens eines filmbildenden Mittels;
- 0,1% bis 3% Gelbildner;
- 0,01% bis 1,5% Chelatbildner;
- 0,1% bis 10% eines Benetzungsmittels; und
- 0,1% bis 20% eines Emollients;
- 0,01% bis 3% Konservierungsmittel.

17. Zusammensetzung gemäß einem der Ansprüche 12 bis 14, **dadurch gekennzeichnet, dass** sie in Wasser umfasst:
- 0,1% bis 0,3% eines Naphthoesäurederivates;
- 1% bis 10% wenigstens eines filmbildenden Mittels;
- 0,1% bis 3% Gelbildner;
- 0,01% bis 1,5% Chelatbildner;
- 0,1% bis 10% eines Benetzungsmittels;
- 0,1% bis 20% eines Emollients;
- 0,1% bis 30% Fettphase;
- 0,01% bis 3% Konservierungsmittel;
- 0 bis 10% Emulgatoren.

18. Zusammensetzung gemäß einem der vorhergehenden Ansprüche zur Verwendung als Medikament.

19. Verfahren zur Herstellung einer Zusammensetzung gemäß einem der Ansprüche 1 bis 17, **dadurch gekennzeichnet, dass** das Verfahren den Schritt des Mischens eines physiologisch verträglichen Trägers, umfassend wenigstens ein Naphthoesäurederivat, mit wenigstens einem filmbildenden Mittel umfasst, um eine Zusammensetzung zu erhalten, in der besagtes Naphthoesäurederivat in dispergierter Form in besagter Zusammensetzung vorliegt.

20. Verfahren zur Herstellung gemäß Anspruch 19, **dadurch gekennzeichnet, dass** das Verfahren die folgenden Schritte umfasst:
a) Mischen des Naphthoesäurederivates mit wenigstens einem Benetzungsmittel und wenigstens einem Chelatbildner in Wasser, bis besagtes Naphthoesäurederivat vollständig dispergiert ist, um die wässrige aktive Phase zu erhalten;
b) Mischen wenigstens eines filmbildenden Mittels mit Wasser, um eine filmbildende Phase zu erhalten;
c) Einleiten der in b) erhaltenen filmbildenden Phase in die in a) erhaltene wässrige aktive Phase oder umgekehrt, um eine wässrige Zusammensetzung zu erhalten.

21. Verfahren zur Herstellung gemäß Anspruch 20, **dadurch gekennzeichnet, dass** das Verfahren außerdem am Ende von Schritt c) einen Schritt des Mischens der in c) erhaltenen wässrigen Zusammensetzung mit wenigstens einer Fettphase umfasst, um eine Emulsion zu erhalten.

22. Verwendung einer Zusammensetzung gemäß einem der Ansprüche 1 bis 17 zur Herstellung einer pharmazeutischen Zusammensetzung zur Behandlung und/oder Prävention dermatologischer Erkrankungen, die mit einer Störung der Keratinisierung im Zusammenhang mit der Zelldifferenzierung und Zellproliferation verbunden sind und aus Acne vulgaris, Acne comedonica, Acne papulopustulosa, Acne papulocomedonica, Acne nodulocystica, Acne conglobata, Acne scleroticans nuchae, rezidivierende Acne miliaris, Acne necrotica, Acne neonatorum, Acne professionalis, Acne rosaceae, Acne senilis, Acne solaris und Acne medikamentosa ausgewählt sind.

23. Verwendung einer Zusammensetzung gemäß Anspruch 22 zur Herstellung einer pharmazeutischen Zusammensetzung zur Prävention und/oder Behandlung von Acne vulgaris.

24. Kosmetische Verwendung einer Zusammensetzung gemäß einem der Ansprüche 1 bis 17 zur Bekämpfung des fettigen Aussehens von Haut oder Haaren, bei der Behandlung physiologisch fetter Hautbereiche oder zur Prävention und/oder Bekämpfung der lichtinduzierten oder chronologischen Alterung.

## Claims

1. A composition comprising, into a physiologically acceptable medium, at least one naphthoic acid derivative and at least one film-forming agent, **characterized in that** the naphthoic acid derivative is in a form dispersed into said composition and is a compound of formula (I): 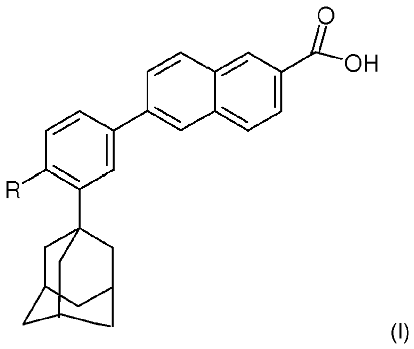 where R is a hydrogen atom, a hydroxyl radical, a branched or unbranched alkyl radical having from 1 to 4 carbon atoms, an alkoxy radical having from 1 to 10 carbon atoms or a cycloaliphatic radical,
and **in that** the film-forming agent is selected among polyvinylpyrrolidones, vinyl acetate derivatives, polyvinyl alcohols, cyanoacrylic polymers, acrylic/methacrylic and polymethacrylate/butylacrylate copolymers and sodium hyaluronate.

2. The composition according to claim 1, **characterized in that** the concentration of the naphthoic acid derivative is between 0.001 % and 10%, preferably between 0.01 % and 5%, more preferably between 0.05% and 2% by weight of the total weight of the composition.

3. The composition according to one of the previous claims, **characterized in that** the naphthoic acid derivative is selected among 6-[3-(1-adamantyl)-4-methoxyphenyl]-2-naphthoic acid, 6-[3-(1-adamantyl)-4-hydroxyphenyl]-2-naphthoic acid, 6-[3-(1-adamantyl)-4-decyloxyphenyl]-2-naphthoic acid and 6-[3-(1-adamantyl)-4-hexyloxyphenyl]-2-naphthoic acid.

4. The composition according to one of claims 1 to 3, **characterized in that** the naphthoic acid derivative is 6-[3-(1-adamantyl)-4-methoxyphenyl]-2-naphthoic acid.

5. The composition according to claim 4, **characterized in that** the concentration of 6-[3-(1-adamantyl)-4-methoxyphenyl]-2-naphthoic acid is about 0.1% by weight relative to the total weight of the composition.

6. The composition according to claim 4, **characterized in that** the concentration of 6-[3-(1-adamantyl)-4-methoxyphenyl]-2-naphthoic acid is about 0.3% by weight relative to the total weight of the composition.

7. The composition according to one of the previous claims, **characterized in that** the film-forming agent is selected among sodium hyaluronate and copovidone.

8. The composition according to one of the previous claims, **characterized in that** the concentration of film-forming agent is between 0.5% and 20% by weight relative to the total weight of the composition, preferably between 1% and 10%.

9. The composition according to claim 8, **characterized in that** the concentration of film-forming agent is 2%, 4% or 6% by weight of the total weight of the composition.

10. The composition according to one of the previous claims, **characterized in that** it is in the form of an aqueous, aqueous-alcoholic or oily dispersion, an aqueous, anhydrous or lipophilic gel, an emulsion of liquid, semi-liquid or solid consistency, obtained by dispersing a fatty phase in an aqueous phase or vice versa, or a suspension of soft, semi-liquid or solid consistency, or a microemulsion, microcapsules, microparticles or vesicular dispersion of ionic and/or nonionic type.

11. The composition according to claim 10, **characterized in that** it is in the form of a gel.

12. The composition according to claim 10, **characterized in that** it is in the form of a cream.

13. The composition according to claim 10, **characterized in that** it is in the form of a lotion.

14. The composition according to claim 10, **characterized in that** it is in the form of a cream-gel.

15. The composition according to claim 11, **characterized by** comprising, in water:
from 0.1% to 0.3% of a naphthoic acid derivative;
from 1% to 10% of at least one film-forming agent;
from 0.1% to 3% of gelling agents;
from 0.01% to 1.5% of chelating agents;
from 0.1% to 10% of a wetting agent; and
from 0.01% to 3% of preservatives.

16. The composition according to claim 11, **characterized by** comprising, in water:
from 0.1% to 0.3% of a naphthoic acid derivative;
from 1% to 10% of at least one film-forming agent;
from 0.1% to 3% of gelling agents;
from 0.01% to 1.5% of chelating agents;
from 0.1% to 10% of a wetting agent;
from 0.1% to 20% of an emollient; and
from 0.01% to 3% of preservatives.

17. The composition according to one of claims 12 to 14, **characterized by** comprising, in water:
from 0.1% to 0.3% of a naphthoic acid derivative;
from 1% to 10% of at least one film-forming agent;
from 0.1% to 3% of gelling agents;
from 0.01% to 1.5% of chelating agents;
from 0.1 % to 10% of a wetting agent;
from 0.1% to 20% of an emollient;
from 0.1 % to 30% of fatty phase;
from 0.01% to 3% of preservatives;
from 0 to 10% of emulsifiers.

18. The composition according to any one of the previous claims, for use as a medicament.

19. A process for preparing a composition according to any one of claims 1 to 17, **characterized by** comprising the step of mixing a physiologically acceptable vehicle which comprises at least one naphthoic acid derivative with at least one film-forming agent, to obtain a composition in which said naphthoic acid derivative is in a form dispersed in said composition.

20. The process for preparing according to claim 19, **characterized by** comprising the following steps:
a) mixing the naphthoic acid derivative with at least one wetting agent, and at least one chelating agent in water, until the said naphthoic acid derivative is fully dispersed, to obtain the aqueous active phase;
b) mixing the at least one film-forming agent with water to obtain a film-forming phase;
c) introducing the film-forming phase obtained in b) into the aqueous active phase obtained in a), or vice versa, to obtain an aqueous composition.

21. The process for preparing according to claim 20, **characterized by** further comprising, at the end of step c), a step of mixing the aqueous composition obtained in c) with at least one fatty phase to obtain an emulsion.

22. Use of a composition according to one of claims 1 to 17 for the preparation of a pharmaceutically composition for treating and/or preventing dermatological complaints associated with a keratinization disorder relating to cell differentiation and proliferation, selected from common acne, comedonal acne, papulopustular acne, papulocomedonal acne, nodulocystic acne, acne conglobata, acne keloid of the nape, recurrent military acne, acne necrotica, acne neonatorum, occupational acne, acne rosacea, senile acne, solar acne and acne medicamentosa.

23. Use of a composition according to claim 22 for the preparation of a pharmaceutically composition for the prevention and/or treatment of common acne.

24. Cosmetic use of a composition according to any one of claims 1 to 17, in order to combat the greasy appearance of the skin or hair in the treatment of physiologically greasy skins, or to prevent and/or combat photo-induced or chronological ageing.
